(19) Europäisches Patentamt
European Patent Office
Office européen des brevets

(11) **EP 0 914 601 B1**

(12) **EUROPÄISCHE PATENTSCHRIFT**

(45) Veröffentlichungstag und Bekanntmachung des Hinweises auf die Patenterteilung:
**08.05.2002   Patentblatt 2002/19**

(51) Int Cl.⁷: **G01N 21/31**, A61B 5/00

(21) Anmeldenummer: **97930288.2**

(86) Internationale Anmeldenummer:
**PCT/CH97/00282**

(22) Anmeldetag: **24.07.1997**

(87) Internationale Veröffentlichungsnummer:
**WO 98/04903 (05.02.1998 Gazette 1998/05)**

(54) **VERFAHREN ZUR NICHTINVASIVEN BESTIMMUNG DER SAUERSTOFFSÄTTIGUNG IN DURCHBLUTETEM GEWEBE**

METHOD OF NON-INVASIVE DETERMINATION OF OXYGEN SATURATION IN TISSUE IN WHICH BLOOD IS CIRCULATING

PROCEDE DE DETERMINATION NON INVASIVE DE LA SATURATION EN OXYGENE DE TISSUS VASCULARISES

(84) Benannte Vertragsstaaten:
**CH DE ES FR GB IT LI**

(30) Priorität: **26.07.1996   CH 186496**

(43) Veröffentlichungstag der Anmeldung:
**12.05.1999   Patentblatt 1999/19**

(73) Patentinhaber: **Linde Medical Sensors AG
4051 Basel (CH)**

(72) Erfinder:
• **SCHÖLLERMANN, Hans
/ (DE)**
• **EBERHARD, Patrick
CH-4123 Allschwil (CH)**

(74) Vertreter: **Bollhalder, Renato et al
Braun Héritier Eschmann AG
Patentanwälte VSP
Holbeinstrasse 36-38
Postfach 160
4003 Basel (CH)**

(56) Entgegenhaltungen:
EP-A- 0 303 502          EP-A- 0 335 356
WO-A-94/03102          DE-A- 4 429 758
US-A- 5 216 598          US-A- 5 278 627

• J.M. SCHMITT: "SIMPLE PHOTON DIFFUSION ANALYSIS OF THE EFFECTS OF MULTIPLE SCATTERING ON PULSE OXIMETRY" IEEE TRANSACTIONS ON BIO-MEDICAL ELECTRONICS., Bd. 38, Nr. 12, Dezember 1991, NEW YORK US, Seiten 1194-1203, XP000276077

**Beschreibung**

Die Erfindung betrifft ein Verfahren zur nichtinvasiven Bestimmung der Sauerstoffsättigung in durchblutetem Gewebe, wie es im Oberbegriff des unabhängigen Patentanspruchs 1 definiert ist.

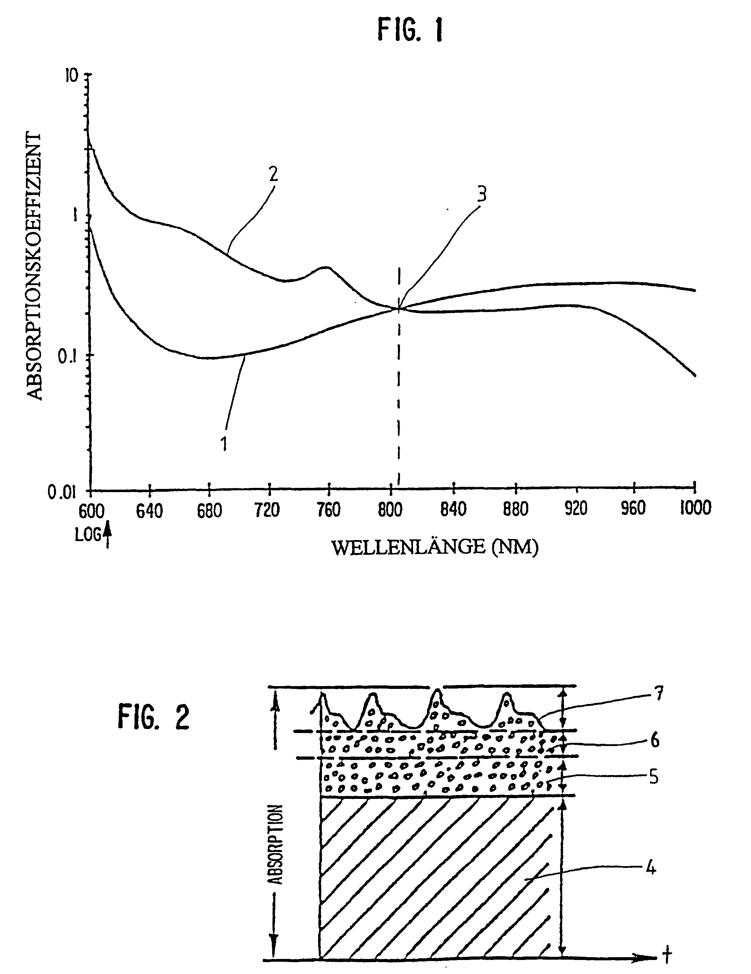

Die optische Messung der Sauerstoffsättigung des Hämoglobins in durchblutetem Gewebe ist seit 1932 bekannt und fand als nichtinvasive Methode zur Ermittlung der Sauerstoffversorgung von lebendem Gewebe in der physiologischen Forschung frühzeitig grosses Interesse. Geräte, die zur Bestimmung dieses Parameters entwickelt wurden, verwendeten ursprünglich Messverfahren, die den spektrophotometrischen Methoden ähnelten, wobei als Messobjekt häufig das Ohrläppchen verwendet wurde. Dieses wurde ähnlich wie eine Küvette mit Licht verschiedener Wellenlängen durchstrahlt. Aus den gemessenen Intensitäten des durchgelassenen Lichts wurde die Sauerstoffsättigung berechnet. Grundlagen dieser Berechnungen waren die bekannten Absorptionsspektren des Hämoglobins in seiner mit Sauerstoff gesättigten Form (Oxyhämoglobin, Fig. 1, Spektrum 1) und seiner sauerstoffreien Form (reduziertes Hämoglobin, Fig. 1, Spektrum 2). Eine Schwierigkeit bei diesem als Ohroxymetrie bezeichneten Verfahren bestand darin, dass im Gegensatz zu einer Küvette eine exakte Kalibrierung des optischen Systems nicht möglich war und die Messmethode deshalb relativ ungenau blieb. Es erwies sich als schwierig, den Einflüssen von Gewebedicke, Blutgehalt, Lichtintensitäten und anderen Variablen ausreichend Rechnung zu tragen. Zwar wurde unter anderem versucht, durch Anwendung von Druck auf das Gewebe das darin enthaltene Blut vorübergehend zu entfernen und dadurch einen künstlichen Nullpunkt zu erzeugen. Versuche dieser Art erwiesen sich jedoch als unpraktisch und zu ungenau. Auch wurde die Tatsache genutzt, dass die Absorptionskoeffizienten von Oxyhämoglobin und reduziertem Hämoglobin bei einigen Wellenlängen (z.B. 505 nm, 548 nm und 805 nm) gleiche Werte (isosbestische Punkte 3, Fig. 1) aufweisen. Durch Verwendung einer dieser Wellenlängen ergab sich zwar ein von der Sauerstoffsättigung unabhängiger Bezugspunkt, der jedoch allein (ohne eine zweite Bezugsgrösse) nicht ausreichend war. Die Ohroxymetrie wurde in den 60er Jahren weiter verfeinert. So wurden in einem von der Firma Hewlett Packard entwickelten Gerät (HP 47201A Ohroxymeter) acht Wellenlängen verwendet, mit Hilfe derer weitere Bezugspunkte zur Kalibrierung des Messsystems verwendet wurden. Auch diese Methode erwies sich als sehr aufwendig und teuer in der Realisierung. Die Ohroxymetrie fand deshalb niemals Zugang zu klinischen Routineanwendungen und wurde fast ausschliesslich in der physiologischen Forschung und für spezielle experimentelle Fragestellungen, z.B. der Aviatik und anderer Gebiete, eingesetzt.

Erst Anfang der 80er Jahre wurde ein neues Verfahren entwickelt, das unter der Bezeichnung Pulsoxymetrie bekannt wurde, und das sich innerhalb kurzer Zeit als Routinemethode zur Überwachung von Patienten vor allem während Anästhesie und Intensivpflege durchsetzte. Das Prinzip der Pulsoxymetrie beruht darauf, die Absorptionsänderungen im Messobjekt zu erfassen, die durch das pulsförmig einströmende arterielle Blut hervorgerufen werden. Dies ermöglicht die Messung der Sauerstoffsättigung des Hämoglobins im arteriellen Blut (arterielle Sauerstoffsättigung). Wie in Figur 2 dargestellt, setzt sich die Gesamtabsorption von Licht in durchblutetem Gewebe aus folgenden Komponenten zusammen:

- Absorption 4 durch Gewebe und Knochen
- Absorption 5 durch venöses Blut
- Absorption 6 durch arterielles Blut
- Variable Absorption 7 infolge von Volumenänderungen, die durch das pulsierende arterielle Blut erzeugt werden.

Die ersten drei Komponenten sind in der Regel über einen längeren Zeitraum stationär und werden nachfolgend zusammengefasst als DC-Anteil bezeichnet. Nur die vierte Komponente (AC-Anteil) ist synchron zu den Herzkontraktionen periodisch zeitabhängig.

Als Lichtquellen werden in der Pulsoxymetrie in der Regel zwei lichtemittierende Dioden (LED) mit Wellenlängen von etwa 660 nm (rot) und etwa 890 nm (infrarot) verwendet. Wie aus Figur 1 ersichtlich, ist die Lichtabsorption von Hämoglobin bei 660 nm stark vom Sauerstoffgehalt des Blutes abhängig. Dagegen befindet sich die infrarote Wellenlänge in der Nähe des isosbestischen Punktes 3 von 805 nm, so dass nur eine geringe Abhängigkeit der Lichtabsorption vom Sauerstoffgehalt vorliegt. Die Absorption im infraroten Bereich wird deshalb als Bezugsgrösse verwendet. Das von den beiden LED ausgestrahlte Licht wird in einen gut durchbluteten Körperteil (z.B. Fingerbeere, Ohrläppchen oder Zehe) geleitet. Dort wird das Licht mehrfach gestreut und teilweise absorbiert. Das austretende Licht wird mit einer Fotodiode gemessen, die in der Regel den LED gegenüberliegend angeordnet ist. Die beiden LED und die Fotodiode sind üblicherweise in einer Baueinheit integriert, die als pulsoxymetrischer Sensor bezeichnet wird. Die separate Messung des roten und infraroten Lichtes mit nur einer Fotodiode wird durch Verwendung von alternierenden Lichtimpulsen der beiden Wellenlängen ermöglicht, die getrennt messtechnisch erfasst und ausgewertet werden.

Für die pulsoxymetrische Ermittlung der arteriellen Sauerstoffsättigung ($SaO_2^P$) werden die stationären Komponenten ($DC_R$, $DC_{IR}$) und die zeitabhängigen Komponenten ($AC_R$, $AC_{IR}$) der gemessenen roten (R) und infraroten (IR) Lichtintensitäten verwertet. Üblicherweise wird $SaO_2^P$ über die Beziehung

$$SaO_2{}^P = f(Q) \text{ mit } Q = \frac{AC_R}{DC_R} : \frac{AC_{IR}}{DC_{IR}} \tag{1}$$

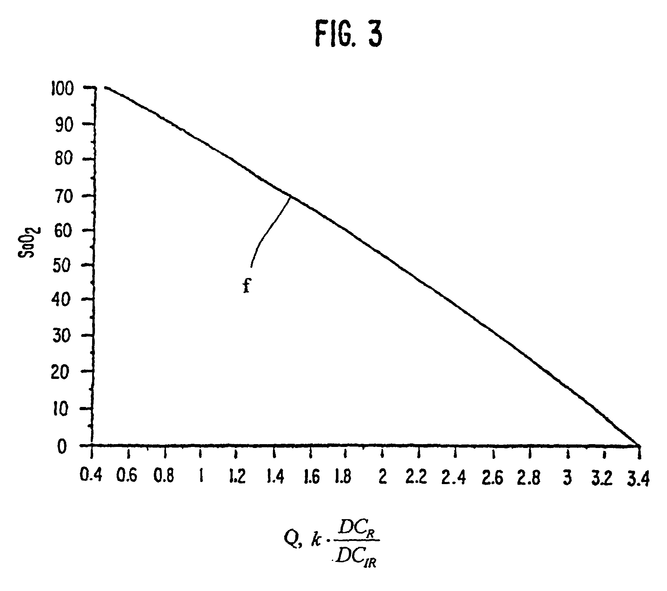

ermittelt, wobei f eine empirisch ermittelte Funktion darstellt (siehe Figur 3), die für ein vorgegebenes Messsytem (Pulsoxymeter und Sensor) unabhängig von Messstelle und Patient ist.

Ein noch nicht zufriedenstellend gelöstes Problem bei der pulsoxymetrischen Messung besteht darin, dass Störungen der Messsignale, die durch Bewegungen des Patienten oder dessen Umgebung verursacht werden, oft nicht vollständig eliminiert werden können. Dies trifft besonders dann zu, wenn während schwacher physiologischer Signale sehr starke bewegungsbedingte Störungen auftreten. So kann es durchaus vorkommen, dass das Verhältnis $AC_R/DC_R$ bzw. $AC_{IR}/DC_{IR}$ kleiner als $10^{-3}$ ist, während die durch Bewegungen bedingten Störsignale hundertfach grösser sind. Da die Frequenzverteilung von Bewegungsartefakten oft diejenige des physiologischen Signals überlappt, ist eine saubere Trennung der Messignale von den Störsignalen durch Filterung in solchen Fällen nicht möglich. Die Ermittlung von $SaO_2$ gemäss Gleichung (1) ist dann mit ausreichender Genauigkeit nicht mehr gewährleistet, so dass erhebliche Messfehler auftreten können. In der Regel werden Störungen dieser Art von der Software des Gerätes erkannt, und der Bediener des Pulsoxymeters wird z.B. durch eine an der Anzeige des Gerätes erscheinende Meldung darauf hingewiesen, dass die Messung nicht mehr möglich ist. Der Bediener des Pulsoxymeters hat nun die Gelegenheit, die Ursache für die Störung zu beseitigen, was jedoch nicht immer durchführbar ist. Besonders problematisch sind Bewegungsstörungen während der Überwachung von Neugeborenen. Der pulsoxymetrische Sensor wird dort zumeist an der Handfläche oder am Fuss des Patienten angebracht. Eine Ruhigstellung der Hand oder des Fusses ist in der Regel nicht möglich. Hinzu kommt, dass gerade an diesen Sensorpositionen das Messsignal oft sehr schwach ist. Aus diesen Gründen tritt bei der Überwachung von Neugeborenen häufig die Situation ein, dass während einer längeren Zeit die Messung der arteriellen Sauerstoffsättigung ausgesetzt werden muss. Dies ist gerade bei Frühgeborenen mit unreifen Lungen kritisch, da bei diesen Patienten rasche und unerwartete Änderungen der arteriellen Sauerstoffsättigung auftreten können, die bei Nichterkennung zu schweren Komplikationen führen können.

Der Erfindung liegt die Aufgabe zugrunde, die Bestimmung der Sauerstoffsättigung in Situationen zu ermöglichen, in denen wegen gleichzeitig vorliegender schwacher physiologischer Signale und starker bewegungsbedingter Störsignale die pulsoxymetrische Methode nicht mehr anwendbar ist.

Diese Aufgabe wird durch das erfindungsgemässe Verfahren gelöst, wie es im unabhängigen Patentanspruch 1 definiert ist. Bevorzugte Ausführungsvarianten ergeben sich aus den abhängigen Patentansprüchen.

Als vorwiegend stationäre Grössen werden die DC-Komponenten des aus dem Gewebe austretenden roten und infraroten Lichtes wesentlich weniger durch bewegungsbedingte Störungen beeinflusst als die Quotienten $AC_R/DC_R$ und $AC_{IR}/DC_{IR}$. Es bietet sich deshalb an, im Fall der oben beschriebenen Störsituationen nur die DC-Komponenten zur Berechnung der Sauerstoffsättigung zu verwenden, so wie dies bei der anfangs beschriebenen Ohroxymetrie durchgeführt wurde. Die grundsätzliche Schwierigkeit der Ohroxymetrie bestand wie oben geschildert darin, dass eine Kalibrierung des Messsystems nicht möglich war, und dass somit die Einflüsse von Gewebedicke, Blutgehalt und anderen Variablen nicht mit ausreichender Genauigkeit und mit einfachen Mitteln berücksichtigt werden konnten.

Dieses Problem wird mit dem erfindungsgemässen Verfahren dadurch gelöst, dass die pulsoxymetrische Messung der Sauerstoffsättigung ($SaO_2{}^P$) zur Kalibrierung der mittels der DC-Komponenten bestimmten Sauerstoffsättigungswerte ($SaO_2{}^{DC}$) verwendet wird. Dem Verfahren liegt die Beobachtung zugrunde, dass zwischen dem in Gleichung (1) definierten Quotienten Q und dem Verhältnis $DC_R/DC_{IR}$ näherungsweise die Beziehung

$$Q = k \cdot \frac{DC_R}{DC_{IR}} \tag{2}$$

gilt, wobei der Faktor k unverändert bleibt, solange sich der pulsoxymetrische Sensor an derselben Messposition befindet und solange keine Änderungen des Blutvolumens und anderer variabler Parameter am Ort der Messung stattfinden. Die Kalibrierung erfolgt dadurch, dass der Faktor k zu einem Zeitpunkt, an dem keine oder nur geringe bewegungsbedingte Störungen vorliegen, gemäss Gleichung (2) ermittelt wird. Danach kann die Sauerstoffsättigung $SaO_2{}^{DC}$ für eine bestimmte Zeit aus den DC-Werten gemäss

$$SaO_2{}^{DC} = f(k \cdot \frac{DC_R}{DC_{IR}}) \tag{3}$$

ermittelt werden. Hierbei ist f dieselbe empirisch ermittelte Funktion gemäss Gleichung (1), die zur Berechnung von $SaO_2{}^P$ verwendet wird.

Es hat sich als zweckmässig erwiesen, den Kalibrierfaktor k während der pulsoxymetrischen Messung fortlaufend anzupassen und im Fall von Störsituationen den zuletzt ermittelten Wert von k zur Berechnung von $SaO_2^{DC}$ zu verwenden. In der Praxis hat sich gezeigt, dass innerhalb eines Zeitraums von 5 bis 10 Minuten keine wesentlichen Änderungen von k auftreten, sofern der Sensor an derselben Messposition verbleibt und keine raschen Blutdruckänderungen stattfinden. Somit können Störsituationen über Zeiten dieser Grössenordnung mit Hilfe des erfindungsgemässen Verfahrens überbrückt werden. Während der Überwachung von Neugeborenen sind diese Zeiten zumeist ausreichend, da heftige Bewegungen der Hände oder Füsse in der Regel durch kurze Ruhepausen unterbrochen werden, während der eine Neubestimmung von k vorgenommen werden kann.

Es versteht sich, dass an Stelle der in Gleichung (2) angegebenen Näherung auch andere, verfeinerte Beziehungen zwischen Q und $DC_R/DC_{IR}$ im Sinne des erfindungsgemässen Verfahrens zur Kalibrierung verwendet werden können, sofern dies im Interesse einer erhöhten Genauigkeit erwünscht ist. Beispielsweise würde sich hierfür ein Polynom der Form

$$Q = \sum_{i=1}^{n} k_i \cdot (\frac{DC_R}{DC_{IR}})^{i-1} \quad \text{mit } n \geq 2 \qquad (4)$$

eignen. Die Kalibrierung wäre jedoch in diesem Fall wesentlich aufwendiger, da dann mehrere Wertepaare von Q und $DC_R/DC_{IR}$ (zumindest n) zur Ermittlung von $k_1,...,k_n$ verwendet werden müssten.

**Patentansprüche**

1. Verfahren zur nichtinvasiven Bestimmung der Sauerstoffsättigung in durchblutetem Gewebe, bei dem das Gewebe mit Licht mindestens zweier verschiedener Wellenlängen durchstrahlt wird und die DC- und AC-Komponenten des austretenden Lichts gemessen werden, **dadurch gekennzeichnet, dass** während eines ersten Zeitintervalls aus den DC- und AC-Komponenten des austretenden Lichts mindestens ein Kalibrierfaktor k, $k_1$ bis $k_n$ ermittelt wird und während eines zweiten Zeitintervalls aus den DC-Komponenten des austretenden Lichts und dem oder den ermittelten Kalibrierfaktoren k, $k_1$ bis $k_n$ die Sauerstoffsättigung im Gewebe fortlaufend berechnet wird.

2. Verfahren nach Anspruch 1, **dadurch gekennzeichnet, dass** der oder die Kalibrierfaktoren k, $k_1$ bis $k_n$ während eines Zeitintervalls ermittelt werden, während dem keine oder nur geringe bewegungsbedingte Störungen vorliegen.

3. Verfahren nach Anspruch 1 oder 2, **dadurch gekennzeichnet, dass** die Ermittlung des oder der Kalibrierfaktoren k, $k_1$ bis $k_n$ periodisch wiederholt wird.

4. Verfahren nach einem der Ansprüche 1 bis 3, **dadurch gekennzeichnet, dass** die Ermittlung des oder der Kalibrierfaktoren k, $k_1$ bis $k_n$ alle 10 Sekunden bis 10 Minuten wiederholt wird.

5. Verfahren nach einem der Ansprüche 1 bis 4, **dadurch gekennzeichnet, dass** die Sauerstoffsättigung $SaO_2^{DC}$ mit der Gleichung $SaO_2^{DC} = f(k \cdot \frac{DC_{L1}}{DC_{L2}})$ berechnet wird, wobei $DC_{L1}$ und $DC_{L2}$ die DC-Komponenten des austretenden Lichts der beiden verwendeten Wellenlängen sind und f eine empirisch ermittelte Funktion ist, die für eine gegebene Messvorrichtung unabhängig von Messstelle und Patient ist.

6. Verfahren nach Anspruch 5, **dadurch gekennzeichnet, dass** der Kalibrierfaktor k mit Hilfe der Gleichung $k \cdot \frac{DC_{L1}}{DC_{L2}} = \frac{AC_{L1}}{DC_{L1}} : \frac{AC_{L2}}{DC_{L2}}$ ermittelt wird, wobei $AC_{L1}$ und $AC_{L2}$ die AC-Komponenten des austretenden Lichts der beiden verwendeten Wellenlängen sind.

7. Verfahren nach einem der Ansprüche 1 bis 4, **dadurch gekennzeichnet, dass** die Sauerstoffsättigung $SaO_2^{DC}$ mit der Gleichung

$$SaO_2^{DC} = f(\sum_{i=1}^{n} k_i \cdot (\frac{DC_{L1}}{DC_{L2}})^{i-1})$$

mit $n \geq 2$ berechnet wird, wobei $DC_{L1}$ und $DC_{L2}$ die DC-Komponenten des austretenden Lichts der beiden verwendeten Wellenlängen sind und f eine empirisch ermittelte Funktion ist, die für eine gegebene Messvorrichtung unabhängig von Messstelle und Patient ist.

8. Verfahren nach Anspruch 7, **dadurch gekennzeichnet, dass** die Kalibrierfaktoren $k_1$ bis $k_n$ mit Hilfe der Gleichung

$$\sum_{i=1}^{n} k_i \cdot (\frac{DC_{L1}}{DC_{L2}})^{i-1} = \frac{AC_{L1}}{DC_{L1}} : \frac{AC_{L2}}{DC_{L2}}$$

ermittelt werden, wobei $AC_{L1}$ und $AC_{L2}$ die AC-Komponenten des austretenden Lichts der beiden verwendeten Wellenlängen sind.

9. Verfahren nach einem der Ansprüche 1 bis 8, **dadurch gekennzeichnet, dass** rotes und infrarotes Licht verwendet wird.

**Claims**

1. Method for the non-invasive determination of the oxygen saturation in tissue supplied with blood, in which the tissue is irradiated with light at at least two different wavelengths and the DC and AC components of the emerging light are measured, **characterized in that**, during a first time interval, at least one calibration factor k, $k_1$ to $k_n$ is determined from the DC and AC components of the emerging light, and, during a second time interval, the oxygen saturation in the tissue is computed progressively from the DC components of the emerging light and the determined calibration factor or calibration factors k, $k_1$ to $k_n$.

2. Method according to Claim 1, **characterized in that** the calibration factor or calibration factors k, $k_1$ to $k_n$ are determined during a time interval during which no or only small movement-conditioned disturbances are present.

3. Method according to Claim 1 or 2, **characterized in that** the determination of the calibration factor or calibration factors k, $k_1$ to $k_n$ is periodically repeated.

4. Method according to one of Claims 1 to 3, **characterized in that** the determination of the calibration factor or calibration factors k, $k_1$ to $k_n$ is repeated every 10 seconds to 10 minutes.

5. Method according to one of Claims 1 to 4, **characterized in that** the oxygen saturation $SaO_2^{DC}$ is computed using the equation $SaO_2^{DC} = f(k \cdot \frac{DC_{L1}}{DC_{L2}})$, where $DC_{L1}$ and $DC_{L2}$ are the DC components of the emerging light of the two wavelengths employed and f is an empirically determined function which, for a given measuring device, is independent of measurement position and patient.

6. Method according to Claim 5, **characterized in that** the calibration factor k is determined with the aid of the equation $k \cdot \frac{DC_{L1}}{DC_{L2}} = \frac{AC_{L1}}{DC_{L1}} : \frac{AC_{L2}}{DC_{L2}}$ where $AC_{L1}$ and $AC_{L2}$ are the AC components of the emerging light at the two wavelengths employed.

7. Method according to one of Claims 1 to 4, **characterized in that** the oxygen saturation $SaO_2^{DC}$ is computed using the equation

$$SaO_2^{DC} = f(\sum_{i=1}^{n} k_i \cdot (\frac{DC_{l,1}}{DC_{l,2}})^{i-1})$$

where $n \geq 2$, where $DC_{L1}$ and $DC_{L2}$ are the DC components of the emerging light of the two wavelengths employed and f is an empirically determined function which, for a given measuring device, is independent of measurement position and patient.

8. Method according to Claim 7, **characterized in that** the calibration factors $k_1$ to $k_n$ are determined with the aid of

the equation

$$\sum_{i=1}^{n} k_i \cdot (\frac{DC_{l,1}}{DC_{l,2}})^{i-1} = \frac{AC_{l,1}}{DC_{l,1}} \cdot \frac{AC_{l,2}}{DC_{l,2}} \ ,$$

where $AC_{L1}$ and $AC_{L2}$ are the AC components of the emerging light of the two wavelengths employed.

**9.**   Method according to one of Claims 1 to 8, **characterized in that** red and infrared light are used.

**Revendications**

**1.**   Procédé de détermination non invasive de la saturation en oxygène de tissus vascularisés, dans lequel on fait passer à travers le tissu une lumière avec au moins deux longueurs d'ondes différentes et que l'on mesure les composantes DC et AC de la lumière transmise, **caractérisé par le fait que** l'on détermine, pendant un premier intervalle de temps, à partir des composantes DC et AC de la lumière transmise, au moins un facteur d'étalonnage k, $k_1$ à $k_n$, et que l'on calcule en continu, pendant un deuxième intervalle de temps, à partir des composantes DC de la lumière transmise et du ou des facteur(s) d'étalonnage k, $k_1$ à $k_n$, la saturation en oxygène dans le tissu.

**2.**   Procédé selon la revendication 1, **caractérisé par le fait que** le ou les facteurs d'étalonnage k, $k_1$ à $k_n$ sont déterminés pendant un intervalle de temps où il n'y a pas ou seulement peu de perturbations dues à des mouvements.

**3.**   Procédé selon la revendication 1 ou 2, **caractérisé par le fait que** la détermination du ou des facteurs d'étalonnage k, $k_1$ à $k_n$ est répétées à des intervalles réguliers.

**4.**   Procédé selon l'une quelconque des revendications 1 à 3, **caractérisé par le fait que** la détermination du ou des facteurs d'étalonnage k, $k_1$ à $k_n$ est répétée toutes les 10 secondes à 10 minutes.

**5.**   Procédé selon l'une quelconque des revendications 1 à 4, **caractérisé par le fait que** la saturation en oxygène $SaO_2^{DC}$ est calculée à l'aide de l'équation suivante :

$$SaO_2^{DC} = f(k \cdot \frac{DC_{L1}}{DC_{L2}})$$

où $DC_{L1}$ et $DC_{L2}$ représentent les composantes DC de la lumière transmise aux deux longueurs d'ondes utilisées et f est une fonction déterminée empiriquement qui, pour un dispositif de mesure donné, est indépendante du patient et de la région de mesure.

**6.**   Procédé selon la revendication 5, **caractérisé par le fait que** le facteur d'étalonnage k est déterminé à l'aide de l'équation suivante :

$$k \cdot \frac{DC_{L1}}{DC_{L2}} = \frac{AC_{L1}}{DC_{L1}} \div \frac{AC_{L1}}{DC_{L2}}$$

dans laquelle $AC_{L1}$ et $AC_{L2}$ sont les composantes AC de la lumière transmise aux deux longueurs d'ondes utilisées.

**7.**   Procédé selon l'une des revendications 1 à 4, **caractérisé par le fait que** l'on calcule la saturation en oxygène $SaO_2^{DC}$ à l'aide de l'équation suivante :

$$SaO_2{}^{DC} = f(\sum_{i=1}^{n} k_i \cdot (\frac{DC_{l,1}}{DC_{l,2}})^{i-1})$$

avec $n \geq 2$, où $DC_{L1}$ et $DC_{L2}$ représentent les composantes DC de la lumière transmise aux deux longueurs d'ondes utilisées et f est une fonction empirique qui, pour un dispositif de mesure donné, est indépendante du patient et de la région de mesure.

8.  Procédé selon la revendication 7, **caractérisé par le fait que** les facteurs d'étalonnage $k_1$ à $k_n$ sont déterminés à l'aide de l'équation suivante :

$$\sum_{i=1}^{n} k_i \cdot (\frac{DC_{l,1}}{DC_{l,2}})^{i-1} = \frac{AC_{l,1}}{DC_{l,1}} \div \frac{AC_{l,2}}{DC_{l,2}}$$

où $AC_{L1}$ et $AC_{L2}$ représentent les composantes AC de la lumière transmise aux deux longueurs d'ondes utilisées.

9.  Procédé selon l'une quelconque des revendications 1 à 8, **caractérisé par le fait que** l'on utilise de la lumière rouge et de la lumière infrarouge.

# FIG. 1

ABSORPTIONSKOEFFIZIENT

10

1

0.1

0.01

600    640    680    720    760    800    840    880    920    960    1000

LOG

WELLENLÄNGE (NM)

FIG. 2

ABSORPTION

## FIG. 3

$$Q,\ k \cdot \frac{DC_R}{DC_{IR}}$$